# EUROPEAN PATENT APPLICATION

(11) **EP 4 516 917 A1**
(43) Date of publication of application: **05.03.2025**
(21) Application number: 23194478.6
(22) Date of filing: 31.08.2023
(51) Int. Cl.: C12P 7/24, C12P 7/04, C12N 9/90, C12P 7/40, C12P 13/04, C12P 19/02

(54) **BIOCATALYTIC ISOMERIZATION OF A CARBOHYDRATE**

(71) Applicant: Cascat GmbH, 94315 Straubing (DE)
(72) Inventor: SIEBER, Volker, 85405 Nandlstadt (DE); WILLER, Vivian Pascal, 94315 Straubing (DE); PICK, André, 36179 Bebra (DE)
(74) Representative: Aera A/S

(57) **Abstract**

A process for converting dihydroxyacetone into glyceraldehyde comprising the step of enzymatically converting dihydroxyacetone into glyceraldehyde using an isomerase that is capable of catalyzing the conversion of dihydroxyacetone into glyceraldehyde. The invention also relates to an isomerase for converting dihydroxyacetone into glyceraldehyde. The isomerase is selected from hydroxypyruvate isomerase, EC 5.3.1.22 and 2-dehydrotetronate isomerase EC 5.3.1.35.

## Description

The present invention relates to a process for converting dihydroxyacetone into glyceraldehyde, an isomerase being capable of catalyzing the conversion of dihydroxyacetone into glyceraldehyde, and the use of an isomerase being capable of catalyzing the conversion of dihydroxyacetone into glyceraldehyde in the conversion of dihydroxyacetone into glyceraldehyde. Besides, the present invention relates to the use of an isomerase being capable of catalyzing the conversion of dihydroxyacetone into glyceraldehyde in the conversion process of dihydroxyacetone to a carbohydrate or to ethanol, isobutanol, alanine, pyruvate, glycerate, valine and/or lactate.

This invention is in the field of carbohydrate biochemistry and more specifically in the field of isomerising dihydroxyacetone (DHA) to glyceraldehyde (GA). The DHA can be derived from CO₂, biomass, fossil hydrocarbons, carbohydrates and glycerol from biodiesel industry and bioconversion of this compound into a target organic compound by an enzymatic process.

DHA is a compound which can be produced in enzymatic reaction cascades, be it top down, that is breaking down complex molecules to smaller products, or bottom up, that is starting e.g. from methane, methanol or even CO₂ to obtain larger molecules. The isomerisation of DHA to GA is in enzymatic reactions an important step.

Carbohydrates are molecules consisting of carbon, hydrogen and oxygen atoms with the empirical formula Cₘ(H₂O)ₙ (where m could be different from n). 1 ,3-dihydroxyacetone (DHA) is the simplest compound related to this definition.

Saccharide is the most commonly used term for carbohydrates in the field of biochemistry. The saccharides are divided into four chemical groups: monosaccharides, disaccharides, oligosaccharides and polysaccharides. In general monosaccharides and disaccharides are referred to as sugars. Monosaccharides include glucose and fructose. Sucrose, the granulated table sugar, is a disaccharide made up of glucose and fructose.

DHA is an isomer of glyceraldehyde (GA) and, together, they are generally referred to as trioses, meaning three-carbon monosaccharides. The L- and D-glyceraldehyde enantiomers are termed aldoses, whereas DHA is named a ketose since it possesses a ketone function.

Carbohydrate synthesis has become a major focus of current biological research. Carbohydrates are structurally complex with several stereoisomers that exhibit specific orientation relative to each other. Depending on the position of the isomers and the relationship between them, several natural and unnatural carbohydrates are identified. The synthesis of such highly functionalized, complex molecules via chemical methods often requires many selective protection and deprotection steps which can be avoided using enzymes. Enzymes are increasingly recognized as useful catalysts for synthesis of complex carbohydrates. One of the main attractions for the use of enzymes is their ability to perform reactions in a stereoselective way. Additionally, the use of enzymes has several advantages over chemical methods. Because of the mild conditions required for enzymatic reactions and the regioselectivity displayed by the enzymes unprotected substrates can be used. Since most enzymes operate at room temperature in aqueous solution around pH 7, the reactions by multiples enzymes are often compatible with each other. This makes it possible to combine several enzymes in a one-pot multistep reaction sequence. Their use in aqueous solution and their biodegradability make enzymes also an excellent environmentally acceptable options. The high regio- and stereoselectivity and catalytic efficiency make enzymes especially useful for the synthesis of complex and highly functionalized carbohydrates.

Besides, a number of technical processes have been described that use isolated enzymes for the production of chemicals. For example, alcohol dehydrogenases are used in the production of chiral alcohols from ketones. Such processes require cofactor (NAD) regeneration which can be achieved, for example, by adding glucose and glucose dehydrogenase. Such processes have been designed to produce high-value chemicals but not to provide enzyme systems comprising multiple enzyme reactions that convert carbohydrates into chemicals with high energy and carbon efficiency.

Zhang et al., 2008 describe the idea for cell free enzymatic conversion of glucose to n-butanol. The concept includes a minimum of 18 enzymes, several different cofactors and coenzymes (e.g. ATP, ADP, NADH, NAD, ferredoxin and coenzyme A). In addition, the postulated process results in a net-production of ATP so that it requires also an ATPase enzyme to remove the ATP. Under practical terms control of ATPase addition while maintaining a balanced ATP level is very difficult to achieve. In summary, the described process would be expensive, technically instable and would give only low butanol yields.

For the preparation of carbohydrates from smaller building blocks or the production of other chemicals sophisticated enzymatic cascades are necessary which in particular can handle the conversion of DHA, which is produced during those cascades, to GA.

The DHA can serve as a starting material for the synthesis of fructose, glucose and mannose as well as other chemicals such as ethanol, isobutanol, lactate, alanine, valine, glycerate, and pyruvate.

Enzymes for the isomerization of unphosphorylated triose substrates, such as DHA, are not known to exist as part of natural pathways. Also, the equilibrium of DHA and GA is composed of 60-65 % DHA and 35-40 % D-glyceraldehyde regardless of the starting point, confirming that DHA is thermodynamically more stable than GA (Bricotte, L., Chougrani, K., Alard, V., Ladmiral, V., & Caillol, S. (2023). Dihydroxyacetone: A User Guide for a Challenging Bio-Based Synthon. Molecules, 28(6), 2724).

The prior art does not teach the isomerisation of unphosphorylated DHA to GA. In addition, cell-free processes are also not taught in the prior art.

Hence, there is a need for an efficient as well as cost effective process for producing glyceraldehyde using dihydroxyacetone as a substrate. Besides, there is a need that the conversion of DHA to GA is compatible with other conversions in order to be applied in a reaction cascade.

It is the object of the present invention to provide an efficient enzymatic conversion of dihydroxyacetone to glyceraldehyde, which can also be used in a reaction cascade. Minimizing the amount and yield of by-products is also an object. Besides, a process being suitable for commercial and large-scale applications is also desirable.

The object is solved by the process for converting dihydroxyacetone into glyceraldehyde according to claim 1, an isomerase being capable of catalyzing the conversion of dihydroxyacetone into glyceraldehyde according to claim 9, the use of an isomerase being capable of catalyzing the conversion of dihydroxyacetone into glyceraldehyde in the conversion of dihydroxyacetone into glyceraldehyde according to claim 12, the use of an isomerase being capable of catalyzing the conversion of dihydroxyacetone into glyceraldehyde in the conversion process of dihydroxyacetone to a carbohydrate according to claim 13 and the use of an isomerase being capable of catalyzing the conversion of dihydroxyacetone into glyceraldehyde in the conversion process of dihydroxyacetone to ethanol, alanine, pyruvate, glycerate, valine, isobutanol and/or lactate according to claim 14.

In a first aspect the present invention relates to a process for converting dihydroxyacetone into glyceraldehyde comprising, preferably consisting of, the step of enzymatically converting dihydroxyacetone into glyceraldehyde using an isomerase that
a) is capable of catalyzing the conversion of dihydroxyacetone into glyceraldehyde.

Further preferred embodiments are described in the dependent claims as well as in the description.

The conversion of dihydroxyacetone is shown in Eq. 1. DHA is subjected to isomerization reaction using an isomerase enzyme to yield GA.

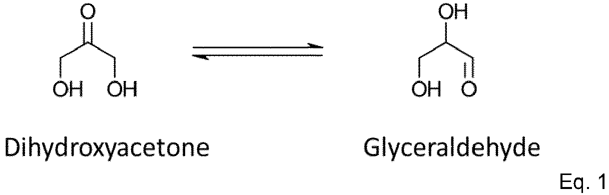

GA occurs in two isomeric forms namely D-glyceraldehyde (DGA) and L-glyceraldehyde (LGA). When talking about GA both isomers are covered.

The enzymes useful in the present invention are either wild-type enzymes or that have undergone certain genetic modifications that improves the reactivity towards non-phosphorylated DHA and GA as substrates. The enzymes with desirable properties, such as ability to use non-phosphorylated substrates, which are derived from the wild type enzymes through recombinant technology are referred as mutants, mutant derivatives or mutants thereof from the corresponding wild type enzymes.

The classification or discussion of a material in any section of this specification as having a particular utility (e.g., catalyst) is for convenience.

Sugars or carbohydrates as used in this invention refer to a biological molecule consisting of carbon, hydrogen and oxygen atoms, usually with hydrogen: oxygen atom ratio of 2:1; in other words with the empirical formula Cₘ(H₂O)ₙ (where m could be different from n).

When referring to a substance occurring as enantiomers such as e.g. D-glyceraldehyde (DGA) and L-glyceraldehyde (LGA) or e.g. L-fructose and D-fructose both isomers of this substance are covered when e.g. the term glyceraldehyde or fructose is used as long as no specific isomer is mentioned.

The citation of references herein does not constitute an admission that such references are prior art or relevant to the patentability of the invention disclosed herein. Any discussion of the contents of the references cited is intended to provide only a general summary of the claims made by the authors of the references and does not constitute an admission as to the correctness of the contents of such references.

While the description and specific examples provide embodiments of the invention, they are for illustrative purposes only and are not intended to limit the scope of the invention. Furthermore, mention of several embodiments having the features mentioned is not intended to exclude other embodiments having additional features or other embodiments having other combinations of the features mentioned. Specific examples are provided to illustrate how the substances and processes of the present invention may be made and used and, unless otherwise expressly stated, are not to be construed as representations that particular embodiments of the present invention have or have not been made or tested.

As used herein, the words preferred and preferably refer to embodiments of the invention that provide certain advantages under certain circumstances. However, other embodiments may also be preferred under the same or different circumstances. Moreover, mention of one or more preferred embodiments does not imply that other embodiments are not useful and is not intended to exclude other embodiments from the scope of the invention.

The DHA applied in the present invention can be derived from various sources.

Sources are e.g. fossil hydrocarbons (including natural gas and shale gas), biogas, biomass and CO₂ released from industrial plants including power generation plants, steel mills and cement factories. Different sources can be used to get access to the precursors of DHA, like syngas and formaldehyde are produced from fossil hydrocarbons, biogas, biomass and CO₂ released form industrial plants. The precursors, formaldehyde and syngas are condensed to produce DHA using chemical catalysts or formaldehyde can be used as substrate in a biocatalytic reaction to produce DHA. The DHA used in this invention can also be derived from glycerol which is produced in large volume as a by-product of biodiesel industry. The DHA used in this invention can also be derived from biological sources such as carbohydrates.

The processes to obtain DHA can be chemical and/or enzymatical. It is preferred according to the present invention that those processes are enzymatical processes.

Using DHA as substrate biocatalytic reactions are employed to form carbohydrates and other chemicals.

Surprisingly, a process for converting dihydroxyacetone into glyceraldehyde comprising the step of enzymatically converting DHA into GA using an isomerase that is capable of catalyzing the conversion of DHA into GA has been found. This process yields GA in high yield and tolerates the presence of other substances as well. Apart of that the process works under mild conditions, that is, without high pressures and temperatures and better tolerates the presence of in-process contaminants. The choice of catalysts makes it possible to obtain a high yield of GA in the process. As a result, a higher proportion based on the substrates can be achieved. The isomerization is faster and leads to higher yields of GA, which allows the reduction in isomerase to be applied.

According to an embodiment the isomerase is capable of catalyzing the conversion of DHA into GA; and comprises alpha keto-acid isomerase activity, preferably beta hydroxy-alpha keto-acid isomerase activity. Surprisingly it was found that isomerases with alpha keto-acid isomerase activity catalyze the conversion of DHA into GA as no natural pathways are known using non-phosphorylated enzymatic steps for the conversion of DHA into GA.

Eq. 2 shows the conversion of a beta hydroxy-alpha keto-acid.

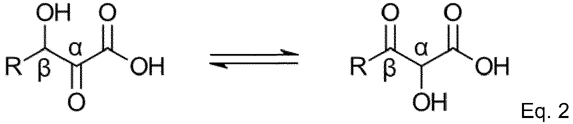

The isomerases which can be used for the conversion of DHA to GA have an alpha keto-acid isomerase activity and preferably beta hydroxy-alpha keto-acid isomerase activity.

According to another embodiment the isomerase is an isomerase belonging to the EC5.3.1 class.

In another embodiment the isomerase is an isomerase belonging to the EC5.3.1.22 class or EC5.3.1.35 class.

In a further embodiment the isomerase is a hydroxypyruvate isomerase or a 2-dehydrotetronate isomerase.

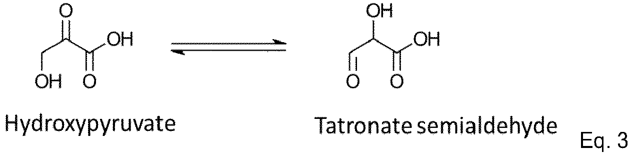

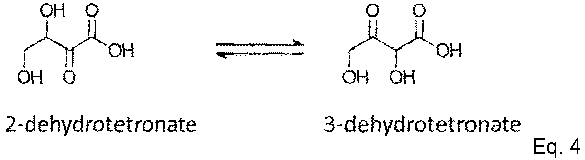

In Eq. 3 the reaction catalyzed by a hydroxypyruvate isomerase and in Eq. 4 the reaction catalyzed by a 2-dehydrotetronate isomerase is shown.

According to an embodiment the isomerase is a hydroxypyruvate isomerase comprising an amino acid sequence that is at least 70 %, preferably at least 80 %, more preferably at least 90 % and even more preferably 95% identical with the sequence according to SEQ ID NO: 35.

According to an embodiment the hydroxypyruvate isomerase has an amino acid sequence according to SEQ ID NO: 5, SEQ ID NO: 27, SEQ ID NO: 29, SEQ ID NO: 31, SEQ ID NO: 33, SEQ ID NO: 35, SEQ ID NO: 39, SEQ ID NO: 37, SEQ ID NO: 45, or SEQ ID NO: 47.

According to an embodiment the isomerase is a 2-dehydrotetronate isomerase comprising an amino acid sequence that is at least 70 %, preferably at least 80 %, more preferably at least 90 % and even more preferably 95% identical with the sequence according to SEQ ID NO: 43.

According to an embodiment the 2-dehydrotetronate isomerase has an amino acid sequence according to SEQ ID NO: 43.

In another embodiment the isomerase is derived from an organism, preferably a microorganism. It is preferred that the organism is *Escherichia coli, Cohaesibacter celericrescens, Polynucleobacter wuianus, Sulfobacillus acidophilus, Sulfobacillus thermosulfidooxidans, Pseudomonas putida, Cupriavidus necator, Vibrio natriegens, Variovorax paradoxus Herbaspirillum seropedicae.*

According to an embodiment the isomerase is a hydroxypyruvate isomerase derived from an organism, preferably a micoorganism. It is preferred that the organism is *Pseudomonas putida.*

According to another embodiment the DHA is in a non-phosphorylated form. It is to be understood that the enzymatical conversion does not require the DHA to be phosphorylated. The use of a non-phosphorylated conversion allows a drastic reduction of required enzymatic steps, no cofactor recycling systems as well as no accumulation of salt load. This makes it possible to have a lean and simple process which also allows an easier down streaming reducing the compounds needed to be separated.

This has the advantage that less by-products occur during the conversion.

In another embodiment the process is cell-free. An easy conversion of DHA into GA allows the implementation of a cell-free process with only a limited number of steps for the generation of valuable carbohydrates and chemicals. No loss of substrate within the metabolism and a straight forward conversion that tolerates higher product concentrations are benefits of a cell-free process.

In an embodiment the process is part of a multi-enzymatic cascade.

The process of the present invention can be carried out in various solvent systems. Preferably it is carried out in aqueous solvent systems.

It is also possible to supplement the aqueous system by a buffer system. Suitable buffers (systems) are known and include conventional buffers (systems), for example, acetate, potassium phosphate, Tris-HCl, glycylglycine and glycine buffers, or mixtures of these. Preferably, a buffer used in a method of the present invention has a pH of 3 - 12, preferably 4 - 12, more preferably 4 - 11. For optimum activity of the biocatalysts ions, e.g. Mg²⁺, to be added. The use of stabilizers, glycerol etc. may allow longer use of biocatalysts.

Preferably the inventive process is carried out at suitable temperatures, e.g. may depend on the enzymes used. Suitable temperatures include 10 - 100 °C, preferably 10 - 90 °C, more preferably 20 - 90 °C, and even more preferably 20 - 80 °C.

Preferably the temperature in step a) is between 10 - 100 °C, more preferably between 20 - 90 °C and even more preferably between 20 - 80 °C.

Further preferred the pH of the composition is between 3-12 and more preferably 4-10.

The inventive process can be used with a limited set of cofactors. In particular, the inventive process does not lead to net ATP production and does not need ATP. Apart of that it is preferred that the inventive process does not use phosphorylative enzyme reactions, and/or uses only enzymes that withstand the inactivating presence of the produced chemicals.

In another aspect the present invention relates to a process for producing a carbohydrate.

The inventive process can be part of a process for the preparation of more complex carbohydrates such as monosaccharides, e.g. pentoses and hexoses. Preferred monosaccharides are fructose, glucose, mannose or a mixture thereof.

The present invention also relates to a process for producing a carbohydrate, preferably a monosaccharide comprising the step of
i) enzymatically converting dihydroxyacetone into glyceraldehyde using an isomerase that is capable of catalyzing the conversion of dihydroxyacetone into glyceraldehyde.

All remarks with respect to the process for converting dihydroxyacetone into glyceraldehyde as mentioned hereinbefore also apply to the process for producing a carbohydrate where applicable.

It is preferred that the monosaccharide is selected from the group consisting of fructose, glucose, mannose or a mixture thereof.

The process further comprises the enzymatical conversion of the GA obtained in step i) into a carbohydrate.

In an embodiment the conversion of the GA into a carbohydrate is an enzymatic reaction cascade wherein at least two enzymes, such as aldolase and isomerase (transaldolase or fructose-6-phosphate aldolase), are used. The mixture of DHA and GA will be converted to fructose and in subsequent steps into glucose or mannose.

In an embodiment the enzymatic conversion is a one-pot reaction.

The process may further comprise additional steps relating to the production of DHA.

The DHA applied in step i) may be produced by a chemical reaction or various chemical reactions. A chemical reaction in the context of the invention is to be understood as a reaction which does not apply enzymes.

Alternatively, the DHA applied in step i) may be produced by enzymatic reaction or various enzymatic reactions.

In an embodiment the DHA is produced starting from a C1-substrate such as methane, methanol or CO₂. This production process may comprise enzymatic and/or chemical reaction steps.

The production of DHA may start from methanol which is converted to DHA in an enzymatic reaction cascade comprising at least three enzymes.

The production of methanol through the conversion of natural gas, coal, CO₂ and biomass has been well documented in patent literature and is known by the person skilled in the art. According to an embodiment the process for the production of a carbohydrate is cell-free.

In one embodiment, the present invention provides a process for producing fructose. In the first stage of this embodiment for producing fructose, DHA is subjected to isomerization reaction using an isomerase enzyme to yield GA.

The process uses the enzyme based DHA isomerization, aldol condensation and isomerization methods; a downstream separation method may be used for the isolation of desired carbohydrates from the undesired co-products or starting materials.

In one embodiment a separation step is used.

The separation step may be a chromatographic based separation process. A non-limiting example of a chromatographic based separation technology is simulated moving bed (SMB) technology. Metal imbedded polystyrene based ion-exchange resins where the metal is the one or more combination of Na⁺, K⁺ and Ca²⁺ have established themselves as the standard in the industry for efficient and economical ketohexose enrichment by SMB. Some non-limiting examples of sugar chromatography ion-exchange resins are Puralite's Chromalite^{®} PCR series ion-exchange resins; Finex^{®} CS11GC, CS12GC and CS16GC ion-exchange resins; Dow Chemicals DOWEX MONOSPHERE^{®} ion-exchange resins.

If e.g. a mixture of ketohexoses/DHA/GA in water is pumped through fixed bed packed with ion-exchange resins in the metal ion form, ketohexoses, being more strongly attracted to the metal on the resin beads, spends more time immobile inside the beads. DHA and GA is attracted less and therefore spends more time outside the beads, in the flowing stream of liquid in the voids between the separation resin beads. The net result is that DHA and GA moves down the resin more rapidly than the ketohexoses. If a stream of liquid is withdrawn from farther down the column, that stream will be enriched in DHA and GA compared to the feed. A stream removed from higher in the column will be enriched in ketohexose. Raffinate typically containing DHA and GA is sent back in the process to be condensed. Extract typically containing ketohexose is further purified by crystallization to obtain 99% pure ketosugar.

The separation process used may also be a fractional crystallization based separation process. If e.g. a mixture of ketohexoses/DHA/GA in water is slowly cooled in the presence of a small amount of ketohexose as a seed catalyst, at appropriate temperature and concentration ketohexoses crystallize out due to its lower solubility in water compared to DHA and GA. In some cases solvents such as ethanol, methanol or butanol may be used to facilitate the crystallization process. Crystals containing mainly ketohexose is filtered out and mother liquor mainly containing DHA and GA is sent back in the process to be condensed. In another method, separation process used is a reverse osmosis separation process. If a mixture of ketohexose/DHA/GA in water is passed across a cellulose acetate (CA) based or poly (vinyl alcohol) (PVA) based membrane, due to the affinity difference between DHA/GA and ketohexoses in water, DHA/GA tend to be more permeable through CA or PVA based membranes compared to ketohexoses. A stream removed from permeate typically containing DHA and GA is sent back in the process to be condensed and the stream removed from retentate typically containing ketohexoses is further purified by crystallization process.

The ketohexoses can also be separated from DHA and GA by converting ketohexose into a calcium ketohexose by treatment with calcium chloride or calcium hydroxide.

Another separation process may be a zeolite based adsorption/desorption method for the separation of ketohexose.

A person skilled in the art may use one or more separation methods as mentioned above for the isolation of ketohexose from DHA and GA.

In another aspect the present invention relates to a process for producing ethanol, alanine, pyruvate, glycerate, valine, isobutanol and/or lactate.

The inventive process can be part of a process for the preparation of ethanol, alanine, pyruvate, glycerate, valine, isobutanol, alanine and/or lactate.

The present invention also relates to a process for producing ethanol, alanine, pyruvate, glycerate, valine, isobutanol, alanine and/or lactate comprising the step of
1) enzymatically converting dihydroxyacetone into glyceraldehyde using an isomerase that is capable of catalyzing the conversion of dihydroxyacetone into glyceraldehyde.

All remarks with respect to the process for converting dihydroxyacetone into glyceraldehyde as mentioned hereinbefore also apply to the to the process for producing ethanol, alanine, pyruvate, glycerate, valine, isobutanol and/or lactate where applicable.

The process further comprises the enzymatical conversion of the GA obtained in step 1) into ethanol, alanine, pyruvate, glycerate, valine, isobutanol, or lactate.

The generated GA will be used to generate glycerate which is converted further into ethanol, isobutanol, alanine, valine, lactate or pyruvate.

In an embodiment the conversion of the GA into ethanol, alanine, pyruvate, glycerate, valine, isobutanol, and/or lactate is an enzymatic reaction cascade wherein at least two, preferably at least three enzymes are used. Using an aldehyde dehydrogenase, dihydroxy acid dehydratase, keto-acid decarboxylase, alanine dehydrogenase, alcohol dehydrogenase, lactate dehydrogenase, and/or acetolactate synthase allows the generation of several chemicals.

In an embodiment the enzymatic conversion is a one-pot reaction.

The process may further comprise additional steps relating to the production of DHA.

The DHA applied in step 1) may be produced by a chemical reaction or various chemical reactions. A chemical reaction in the context of the invention is to be understood as a reaction which does not apply enzymes.

The DHA applied in step 1) may be produced by enzymatic reaction or various enzymatic reactions.

In an embodiment the DHA is produced starting from a C1-substrate such as methane, methanol or CO₂. This production process may comprise enzymatic and/or chemical reaction steps.

The production of DHA may start from methanol which is converted to DHA in an enzymatic reaction cascade comprising at least three enzymes.

In another embodiment the DHA is obtained from a carbohydrate source.

In that case a carbohydrate, preferably a saccharide and more preferably fructose, glucose, mannose or mixtures thereof are converted to DHA. The carbohydrate may be derived from a natural source. For example the carbohydrate may be starch or may be obtained by hydrolysis of biomass, for example in pulp production or in the processing of straw.

This conversion may be a chemical and/or enzymatic conversion. Preferably it is an enzymatic conversion comprising at least two enzymes, preferably at least three enzymes.

According to an embodiment the process for producing ethanol, alanine, pyruvate, glycerate, valine, isobutanol and/or lactate is cell-free.

In another aspect of the present invention relates to an isomerase being capable of catalyzing the conversion of dihydroxyacetone into glyceraldehyde.

All remarks with respect to the process for converting dihydroxyacetone into glyceraldehyde as mentioned hereinbefore also apply to this aspect where applicable.

According to an embodiment the isomerase is capable of catalyzing the conversion of dihydroxyacetone into glyceraldehyde, and comprises alpha keto-acid isomerase activity, preferably beta hydroxy-alpha keto-acid isomerase activity.

In another embodiment the isomerase comprises an amino acid sequence that is at least 70 %, preferably at least 80 %, more preferably at least 90 % and even more preferably at least 95 % identical with the sequence according to SEQ ID NO: 35.

In another embodiment the isomerase comprises an amino acid sequence that is at least 70 %, preferably at least 80 %, more preferably at least 90 % and even more preferably at least 95 % identical with the sequence according to SEQ ID NO: 43.

In a further embodiment the isomerase is an isomerase belonging to the EC5.3.1 class, preferably the isomerase is an isomerase belonging to the EC5.3.1.22 class or EC5.3.1.35 class; and/or the isomerase is a hydroxypyruvate isomerase or a 2-dehydrotetronate isomerase.

According to an embodiment the isomerase has an amino acid sequence according to SEQ ID NO: 5, SEQ ID NO: 27, SEQ ID NO: 29, SEQ ID NO: 31, SEQ ID NO: 33, SEQ ID NO: 35, SEQ ID NO: 39, SEQ ID NO: 37, SEQ ID NO: 43, SEQ ID NO: 45, or SEQ ID NO: 47.

In another aspect the present invention relates to the use of an isomerase being capable of catalyzing the conversion of dihydroxyacetone into glyceraldehyde, preferably being capable of catalyzing the conversion of dihydroxyacetone into glyceraldehyde, and comprising alpha keto-acid isomerase activity, preferably beta hydroxy-alpha keto-acid isomerase activity, in the conversion of dihydroxyacetone into glyceraldehyde.

All remarks with respect to the process for converting dihydroxyacetone into glyceraldehyde and the isomerase as mentioned hereinbefore also apply to this aspect where applicable.

In an embodiment the isomerase is an isomerase belonging to the EC5.3.1 class, preferably the isomerase is an isomerase belonging to the EC5.3.1.22 class or EC5.3.1.35 class; and/or the isomerase is a hydroxypyruvate isomerase or a 2-dehydrotetronate isomerase.

In a further aspect the present invention relates to the use of an isomerase being capable of catalyzing the conversion of dihydroxyacetone into glyceraldehyde, preferably being capable of catalyzing the conversion of dihydroxyacetone into glyceraldehyde, and comprising alpha keto-acid isomerase activity, preferably beta hydroxy-alpha keto-acid isomerase activity, in the conversion process of dihydroxyacetone to a carbohydrate, preferably fructose, glucose, mannose or a mixture thereof.

All remarks with respect to the process for converting dihydroxyacetone into glyceraldehyde, the process for the production of a carbohydrate, the isomerase and the use of the isomerase in the conversion of dihydroxyacetone into glyceraldehyde as mentioned hereinbefore also apply to this aspect where applicable.

In an embodiment the isomerase is an isomerase belonging to the EC5.3.1 class, preferably the isomerase is an isomerase belonging to the EC5.3.1.22 class or EC5.3.1.35 class; and/or the isomerase is a hydroxypyruvate isomerase or a 2-dehydrotetronate isomerase.

In an aspect the present invention relates to the use of an isomerase being capable of catalyzing the conversion of dihydroxyacetone into glyceraldehyde, preferably being capable of catalyzing the conversion of dihydroxyacetone into glyceraldehyde, and comprising alpha keto-acid isomerase activity, preferably beta hydroxy-alpha keto-acid isomerase activity, in the conversion process of dihydroxyacetone to ethanol, alanine, pyruvate, glycerate, valine, isobutanol and/or lactate.

All remarks with respect to the process for converting dihydroxyacetone into glyceraldehyde, the process for producing ethanol, alanine, pyruvate, glycerate, valine, isobutanol and/or lactate, the isomerase and the use of the isomerase in the conversion of dihydroxyacetone into glyceraldehyde as mentioned hereinbefore also apply to this aspect where applicable.

In an embodiment the isomerase is an isomerase belonging to the EC5.3.1 class, preferably the isomerase is an isomerase belonging to the EC5.3.1.22 class or EC5.3.1.35 class; and/or the isomerase is a hydroxypyruvate isomerase or a 2-dehydrotetronate isomerase.

### Brief description of the figures

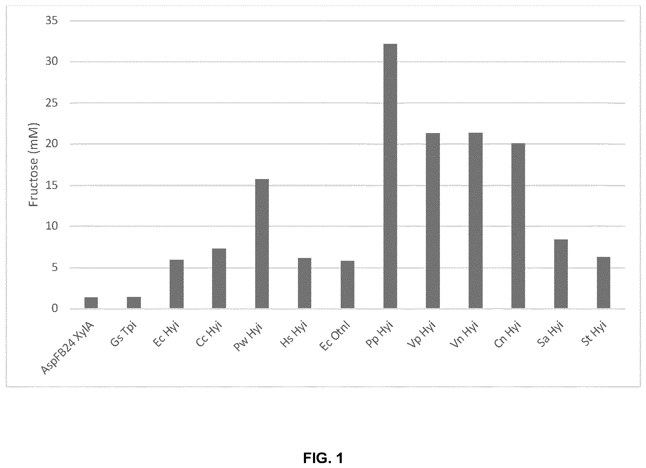

- Fig. 1: shows the comparison of different enzymes for the conversion of dihydroxyacetone into glyceraldehyde after 24h to generate fructose and other monosaccharides.

In the following the abbreviations and the enzymes used in the figure and/or tables are explained:
*Ec*FsaA-A129S - *Escherichia coli* fructose-6-phosphate aldolase variant A129S
*Ec*TalB-F178YR181E - *Escherichia coli* transaldolase B variant F178YR181E
*Ec*YihS - *Escherichia coli* mannose isomerase
*Ec*Otnl - *Escherichia coli* 2-dehydrotetronate isomerase
*Ec*Hyi - *Escherichia coli* hydroxypyruvate isomerase
*Cc*Hyi - *Cohaesibacter celericrescens* hydroxypyruvate isomerase
*Pw*Hyi - *Polynucleobacter wuianus* hydroxypyruvate isomerase
*Sa*Hyi - *Sulfobacillus acidophilus* DSM 10332 hydroxypyruvate isomerase
*St*Hyi - *Sulfobacillus thermosulfidooxidans* DSM 9293 hydroxypyruvate isomerase
PpHyi - *Pseudomonas putida* KT2440 hydroxypyruvate isomerase
*Cn*Hyi - *Cupriavidus necator* H16 hydroxypyruvate isomerase
*Vn*Hyi - *Vibrio natriegens* NBRC 15636 hydroxypyruvate isomerase
*Vp*Hyi - *Variovorax paradoxus* hydroxypyruvate isomerase
*H*sHyi - *Herbaspirillum seropedicae* hydroxypyruvate isomerase
*Asp*FB24XylA- *Arthrobacter sp. FB24* xylose isomerase
*Bs*ALADH - *Bacillus subtilis* alanine dehydrogenase
*Bs*ALS - *Bacillus subtilis* acetolactate synthase
*Gs*LDH - *Geobacillus stearothermophilus* lactate dehydrogenase
*Gs*ADH - *Geobacillus stearothermophilus* alcohol dehydrogenase
*Gs*LEUDH - *Geobacillus stearothermophilus* leucine dehydrogenase
*Gs*Tpi - *Geobacillus stearothermophilus* triose-phosphate isomerase
*Pu*DHT - *Paralcaligenes ureilyticus* DSM 24591 dihydroxy acid dehydratase
*L*/KdcA - *Lactococcus lactis* branched chain keto-acid decarboxylase
ApPDC - *Acetobacter pasteurianus* pyruvate decarboxylase
VpALDH - *Variovorax paradoxus* B4 aldehyde dehydrogenase
PfFLS - *Pseudomonas fluorescens* formolase
GsKARI - *Geobacillus stearothermophilus* ketol-acid reductoisomerase

While the invention has been described with reference to certain embodiments, it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiments, but that the invention will include all embodiments falling within the scope of the appended claims.

### EXPERIMENTAL SECTION

### Materials and Methods

Dihydroxyacetone, methanol, formaldehyde, pyruvate sodium salt, L-alanine, L-valine, lactate, ethanol, isobutanol were obtained commercially from Merck.

Catalase and Alcohol Oxidase were purchased as well from Merck.

### Enzyme production and purification

In brief, a plasmid encoding respective enzyme was used to transform *E. coli* BL21 (DE3).

*Ec*Hyi (SEQ ID NO: 5) was expressed in autoinduction media containing 37 °C for 3 h before shifting the temperature to 16 °C for 21 h. Cell pellet was harvested and kept at -80 °C prior to purification.

*Cc*Hyi (SEQ ID NO: 27) was expressed in autoinduction media containing 37 °C for 3 h before shifting the temperature to 16 °C for 21 h. Cell pellet was harvested and kept at -80 °C prior to purification.

*Pw*Hyi (SEQ ID NO: 29) was expressed in autoinduction media containing 37 °C for 3 h before shifting the temperature to 16 °C for 21 h. Cell pellet was harvested and kept at -80 °C prior to purification.

*Sa*Hyi (SEQ ID NO: 31) was expressed in autoinduction media containing 37 °C for 3 h before shifting the temperature to 16 °C for 21 h. Cell pellet was harvested and kept at -80 °C prior to purification.

*St*Hyi (SEQ ID NO: 33) was expressed in autoinduction media containing 37 °C for 3 h before shifting the temperature to 16 °C for 21 h. Cell pellet was harvested and kept at -80 °C prior to purification.

PpHyi (SEQ ID NO: 35) was expressed in autoinduction media containing 37 °C for 3 h before shifting the temperature to 16 °C for 21 h. Cell pellet was harvested and kept at -80 °C prior to purification.

CnHyi (SEQ ID NO: 39) was expressed in autoinduction media containing 37 °C for 3 h before shifting the temperature to 16 °C for 21 h. Cell pellet was harvested and kept at -80 °C prior to purification.

VnHyi (SEQ ID NO: 37) was expressed in autoinduction media containing 37 °C for 3 h before shifting the temperature to 16 °C for 21 h. Cell pellet was harvested and kept at -80 °C prior to purification.

VpHyi SEQ ID NO: 45) was expressed in autoinduction media containing 37 °C for 3 h before shifting the temperature to 16 °C for 21 h. Cell pellet was harvested and kept at -80 °C prior to purification.

HsHyi SEQ ID NO: 47) was expressed in autoinduction media containing 37 °C for 3 h before shifting the temperature to 16 °C for 21 h. Cell pellet was harvested and kept at -80 °C prior to purification.

EcOtn! (SEQ ID NO: 43) was expressed in autoinduction media containing 37 °C for 3 h before shifting the temperature to 16 °C for 21 h. Cell pellet was harvested and kept at -80 °C prior to purification.

*Ec*FsaA-A129S (SEQ ID NO: 1) was expressed in autoinduction media containing 37 °C for 3 h before shifting the temperature to 16 °C for 21 h. Cell pellet was harvested and kept at -80 °C prior to purification.

*Ec*TalB-F178YR181E (SEQ ID NO: 3) was expressed in autoinduction media containing 37 °C for 3 h before shifting the temperature to 16 °C for 21 h. Cell pellet was harvested and kept at -80 °C prior to purification.

*Ec*YihS (SEQ ID NO: 53) was expressed in autoinduction media containing 37 °C for 3 h before shifting the temperature to 16 °C for 21 h. Cell pellet was harvested and kept at -80 °C prior to purification.

*Pu*DHT (SEQ ID NO: 9) was expressed in TB media and induced using IPTG as described previously (Sutiono. S.; Teshima. M.; Beer. B.; Schenk. G.; Sieber. V. Enabling the Direct Enzymatic Dehydration of D-Glycerate to Pyruvate as the Key Step in Synthetic Enzyme Cascades Used in the Cell-Free Production of Fine Chemicals. ACS Catal. 2020. 10 (5). 3110-3118. doi.org/10.1021/acscatal.9b05068).

*Bs*ALADH (SEQ ID NO: 21) was expressed in autoinduction media containing 37 °C for 3 h before shifting the temperature to 16 °C for 21 h. Cell pellet was harvested and kept at -80 °C prior to purification.

*Gs*LDH (SEQ ID NO: 17) was expressed in autoinduction media containing 37 °C for 3 h before shifting the temperature to 16 °C for 21 h. Cell pellet was harvested and kept at -80 °C prior to purification.

*Gs*ADH (SEQ ID NO: 19) was expressed in autoinduction media containing 37 °C for 3 h before shifting the temperature to 16 °C for 21 h. Cell pellet was harvested and kept at -80 °C prior to purification.

GsTpi (SEQ ID NO: 49) was expressed in autoinduction media containing 37 °C for 3 h before shifting the temperature to 16 °C for 21 h. Cell pellet was harvested and kept at -80 °C prior to purification.

GsKARI (SEQ ID NO: 51) was expressed in autoinduction media containing 37 °C for 3 h before shifting the temperature to 16 °C for 21 h. Cell pellet was harvested and kept at -80 °C prior to purification.

*Ap*PDC (SEQ ID NO. 15) was expressed as described in the literature (Sutiono, S., Satzinger, K., Pick, A., Carsten, J., & Sieber, V. To beat the heat-engineering of the most thermostable pyruvate decarboxylase to date. RSC advances, 2019 9(51), 29743-29746. doi.org/10.1039/C9RA06251C). Cell pellet was harvested and kept at -80 °C prior to purification.

*LI*KdcA (SEQ ID NO: 13) was expressed in autoinduction media at 37 °C for 3 h. before shifting the temperature to 25 °C for 21 h (Sutiono, S., Satzinger, K., Pick, A., Carsten, J., & Sieber, V. To beat the heat-engineering of the most thermostable pyruvate decarboxylase to date. RSC advances, 2019 9(51), 29743-29746. doi.org/10.1039/C9RA06251C). Cell pellet was harvested and kept at -80 °C prior to purification.

*Vp*AIDH (SEQ ID NO: 11) was expressed in autoinduction media containing 37 °C for 3 h before shifting the temperature to 16 °C for 21 h. Cell pellet was harvested and kept at -80 °C prior to purification.

*Asp*FB24XylA (SEQ ID NO: 7) was expressed in autoinduction media containing 37 °C for 3 h before shifting the temperature to 16 °C for 21 h. Cell pellet was harvested and kept at -80 °C prior to purification.

PfFLS (SEQ ID NO: 41) was expressed in autoinduction media containing 37 °C for 3 h before shifting the temperature to 16 °C for 21 h. Cell pellet was harvested and kept at -80 °C prior to purification. The enzyme is described in literature (Siegel, J. B., Smith, A. L., Poust, S., Wargacki, A. J., Bar-Even, A., Louw, C., Baker, D. Computational protein design enables a novel one-carbon assimilation pathway. Proceedings of the National Academy of Sciences, 2015. 112(12), 3704-3709. doi.org/10.1073/pnas.1500545112).

All enzymes expressed harboring hexahistidine in their N-terminal or C-terminal.

For purification the cell pellet was first disrupted using sonicator at 80% and 0.5 s cycle. The cell debris was cleared out by means of centrifugation. All enzymes were then purified using Äkta purifier using His-Trap column FF Crude 5 mL (GE Healthcare. Germany). The buffer was then changed to 50 mM TRIS-HCl or HEPES pH 8.0 using HiPrep desalting column 26/10 50mL (GE Healthcare. Germany). All enzyme was flash frozen in liquid N₂ prior to storage at -80°C until further use.

Samples were analysed using HPLC (Ultimate-3000 HPLC system (Dionex, Idstein, Germany)), equipped with autosampler and a diode-array detector. Chromatographic separation of pyruvate and glycerate was achieved on a Metrosep A Supp10-250/40 column (250 mm, particle size 4.6 mm; Metrohm, Filderstadt, Germany) at 65 °C by isocratic elution with 12 mm ammonium bicarbonate (pH 10), followed by a washing step with 30 mm sodium carbonate (pH 10.4). Mobile phase flow was adjusted to 0.2 mL min⁻¹. 2,3-dihydroxyisovalerate and 2-ketoisovalerate were separated using a Nucleogel Sugar 810H column (300 mm, 7.8 mm internal diameter; Macherey-Nagel, Düren, Germany) at 60 °C by isocratic elution with 3 mm H₂SO₄ (pH 2.2). Mobile phase flow was adjusted to 0.6 mL min⁻¹. System calibration was performed using external standards of each of the abovementioned intermediates. Samples were prepared by filtration (10 kDa MWCO, modified PES; VWR, Darmstadt, Germany) and diluted.

### Biotransformation of Methanol to Dihydroxyacetone (DHA)

The cell-free bioproduction of dihydroxyacetone was performed in 500 µl scale in a 50 ml tube under aerobic conditions. The solution contained the combination of enzymes as shown in Table 1. 150 mM Methanol, 0.5 mM TPP and 5 mM MgCl₂ in 100 mM HEPES pH 7.5 at 25°C. The reaction was carried out in triplicates. The formation of dihydroxyacetone was followed over time by withdrawing 20 µl of aliquot at certain time intervals.

The sample was diluted 25-fold using 2.5 mM H₂SO₄ prior to filtration through 10 KDa spin column (VWR. Germany). The filtrate was analyzed by HPLC using an ion-exclusion column (RezexROA-Organic Acid H+(8%. Phenomenex. Germany) run isocratically using 2.5 mM H₂SO₄ at 70 °C for 20 min.

**Table 1: Enzymes used for biotransformation of methanol to dihydroxyacetone**

| Enzyme | Protein concentration (mg/ml) |
|---|---|
| AOX | 0.1 |
| Catalase | 0.02 |
| Formolase | 13.2 |

**Table 2: Conversion of methanol into dihydroxyacetone**

| Time (h) | Methanol (mM) | Dihydroxyacetone (mM) |
|---|---|---|
| 0 | 150 | 0.0 |
| 3 | 112.2 | 11.4 |
| 7 | 82.3 | 23.2 |
| 24 | 5.0 | 46.5 |

### Biotransformation of Dihydroxyacetone to D-Fructose/D-Glucose

The cell-free bioproduction of fructose/glucose was performed in 1000 µl scale in Eppendorf tube. The solution contained the combination of enzymes as shown in Table 3, 5, 7, 9, 11, 13, 15, 17, 19, 21 or 23. 10.0 mM MgCl₂ and 1.0 mM MnCl₂ and 1.0 mM TPP and 25-50 mM TRIS-HCl or HEPES pH 8.0. and 100 mM DHA at 25°C. The reaction was carried out in triplicates. The formation of fructose, glucose was followed over time by withdrawing 100 µl of aliquot at certain time intervals.

The sample was diluted 25-fold using 5 mM H₂SO₄ prior to filtration through 10 KDa spin column (VWR. Germany). The filtrate was analyzed by HPLC using an ion-exclusion column (RezexROA-Organic Acid H+(8%. Phenomenex. Germany) run isocratically using 2.5 mM H₂SO₄ at 70 °C for 20 min.

**Table 3: Enzymes used for biotransformation of dihydroxyacetone to D-fructose/D-glucose**

| Enzyme | Protein concentration (mg/ml) |
|---|---|
| *Ec*Hyi | 1.2 |
| *Ec*TalB-F178YR181E | 2.5 |
| *Asp*FB24XylA | 0.5 |

**Table 4: Conversion of dihydroxyacetone to D-fructose/D-glucose**

| Time (h) | Dihydroxyacetone (mM) | D-Fructose (mM) | D-Glucose (mM) |
|---|---|---|---|
| 0 | 98.3 | 0.0 | 0.0 |
| 3 | 95.1 | 1.2 | 0.0 |
| 7 | 91.09 | 3.4 | 1.4 |
| 24 | 82.15 | 5.96 | 3.00 |

**Table 5: Enzymes used for biotransformation of dihydroxyacetone to D-fructose/D-glucose**

| Enzyme | Protein concentration (mg/ml) |
|---|---|
| CcHyi | 1.2 |
| *Ec*TalB-F178YR181E | 2.5 |
| *Asp*FB24XylA | 0.5 |

**Table 6: Conversion of dihydroxyacetone to D-fructose/D-glucose**

| Time (h) | Dihydroxyacetone (mM) | Fructose (mM) | Glucose (mM) |
|---|---|---|---|
| 0 | 99.4 | 0.0 | 0.0 |
| 3 | 91.2 | 1.9 | 0.0 |
| 7 | 88.2 | 3.04 | 1.25 |
| 24 | 74.78 | 7.33 | 3.3 |

**Table 7: Enzymes used for biotransformation of dihydroxyacetone to D-fructose/D-glucose**

| Enzyme | Protein concentration (mg/ml) |
|---|---|
| PwHyi | 1.2 |
| *Ec*TalB-F178YR181E | 2.5 |
| *Asp*FB24XylA | 0.5 |

**Table 8: Conversion of dihydroxyacetone to D-fructose/D-glucose**

| Time (h) | Dihydroxyacetone (mM) | Fructose (mM) | Glucose (mM) |
|---|---|---|---|
| 0 | 98.3 | 0.0 | 0 |
| 3 | 95.1 | 3.05 | 0.72 |
| 7 | 81.48 | 5.48 | 1.21 |
| 24 | 64.82 | 15.75 | 2.57 |

**Table 9: Enzymes used for biotransformation of dihydroxyacetone to D-fructose/D-glucose**

| Enzyme | Protein concentration (mg/ml) |
|---|---|
| SaHyi | 1.2 |
| *Ec*TalB-F178YR181E | 2.5 |
| *Asp*FB24XylA | 0.5 |

**Table 10: Conversion of dihydroxyacetone to D-fructose/D-glucose**

| Time (h) | Dihydroxyacetone (mM) | Fructose (mM) | Glucose (mM) |
|---|---|---|---|
| 0 | 98.3 | 0.0 | 0.0 |
| 3 | 95.8 | 1.53 | 0.0 |
| 7 | 90.7 | 3.12 | 0.54 |
| 24 | 79.8 | 8.45 | 0.97 |

**Table 11: Enzymes used for biotransformation of dihydroxyacetone to D-fructose/D-glucose**

| Enzyme | Protein concentration (mg/ml) |
|---|---|
| StHyi | 1.2 |
| *Ec*TalB-F178YR181E | 2.5 |
| *Asp*FB24XylA | 0.5 |

**Table 12: Conversion of dihydroxyacetone to D-fructose/D-glucose**

| Time (h) | Dihydroxyacetone (mM) | Fructose (mM) | Glucose (mM) |
|---|---|---|---|
| 0 | 98.3 | 0.0 | 0.0 |
| 3 | 94.7 | 1.34 | 0.0 |
| 7 | 92.9 | 2.34 | 0.43 |
| 24 | 83.2 | 6.32 | 0.74 |

**Table 13: Enzymes used for biotransformation of dihydroxyacetone to D-fructose/D-glucose**

| Enzyme | Protein concentration (mg/ml) |
|---|---|
| PpHyi | 1.2 |
| *Ec*TalB- F178YR181E | 2.5 |
| *Asp*FB24XylA | 0.5 |

**Table 14: Conversion of dihydroxyacetone to D-fructose/D-glucose**

| Time (h) | Dihydroxyacetone (mM) | Fructose (mM) | Glucose (mM) |
|---|---|---|---|
| 0 | 98.3 | 0.0 | 0.0 |
| 3 | 91.58 | 5.99 | 0.64 |
| 7 | 77.91 | 11.58 | 0.93 |
| 24 | 20.17 | 32.15 | 1.35 |

**Table 15: Enzymes used for biotransformation of dihydroxyacetone to D-fructose/D-glucose**

| Enzyme | Protein concentration (mg/ml) |
|---|---|
| CnHyi | 1.2 |
| *Ec*TalB-F178YR181E | 2.5 |
| *Asp*FB24XylA | 0.5 |

**Table 16: Conversion of dihydroxyacetone to D-fructose/D-glucose**

| Time (h) | Dihydroxyacetone (mM) | Fructose (mM) | Glucose (mM) |
|---|---|---|---|
| 0 | 99.6 | 0.0 | 0.0 |
| 3 | 79.32 | 4.28 | 0.56 |
| 7 | 69.64 | 8.63 | 1.32 |
| 24 | 40.27 | 20.05 | 2.53 |

**Table 17: Enzymes used for biotransformation of dihydroxyacetone to D-fructose/D-glucose**

| Enzyme | Protein concentration (mg/ml) |
|---|---|
| VpHyi | 1.2 |
| *Ec*TalB-F178YR181E | 2.5 |
| *Asp*FB24XylA | 0.5 |

**Table 18: Conversion of dihydroxyacetone to D-fructose/D-glucose**

| Time (h) | Dihydroxyacetone (mM) | Fructose (mM) | Glucose (mM) |
|---|---|---|---|
| 0 | 99.2 | 0.0 | 0.0 |
| 3 | 71.45 | 5.32 | 1.27 |
| 7 | 65.32 | 10.13 | 2.21 |
| 24 | 42.84 | 21.28 | 3.52 |

**Table 19: Enzymes used for biotransformation of dihydroxyacetone to D-fructose/D-glucose**

| Enzyme | Protein concentration (mg/ml) |
|---|---|
| HsHyi | 1.2 |
| *Ec*TalB-F178YR181E | 2.5 |
| *Asp*FB24XylA | 0.5 |

**Table 20: Conversion of dihydroxyacetone to D-fructose/D-glucose**

| Time (h) | Dihydroxyacetone (mM) | Fructose (mM) | Glucose (mM) |
|---|---|---|---|
| 0 | 97.9 | 0.0 | 0.0 |
| 3 | 89.56 | 1.87 | 0.0 |
| 7 | 85.63 | 2.98 | 0.73 |
| 24 | 81.23 | 6.14 | 0.56 |

**Table 21: Enzymes used for biotransformation of dihydroxyacetone to D-fructose/D-glucose**

| Enzyme | Protein concentration (mg/ml) |
|---|---|
| VnHyi | 1.2 |
| *Ec*FsaA-A129S | 2.5 |
| *Asp*FB24XylA | 0.5 |

**Table 22: Conversion of dihydroxyacetone to D-fructose/D-glucose**

| Time (h) | Dihydroxyacetone (mM) | Fructose (mM) | Glucose (mM) |
|---|---|---|---|
| 0 | 97.9 | 0.0 | 0.0 |
| 3 | 78.9 | 4.12 | 0.89 |
| 7 | 75.6 | 8.67 | 1.58 |
| 24 | 44.7 | 21.31 | 4.78 |

**Table 23: Enzymes used for biotransformation of dihydroxyacetone to D-fructose/D-glucose**

| Enzyme | Protein concentration (mg/ml) |
|---|---|
| EcOtnI | 1.2 |
| *Ec*FsaA-A129S | 2.5 |
| *Asp*FB24XylA | 0.5 |

**Table 24: Conversion of dihydroxyacetone to D-fructose/D-glucose**

| Time (h) | Dihydroxyacetone (mM) | Fructose (mM) | Glucose (mM) |
|---|---|---|---|
| 0 | 97.9 | 0.0 | 0.0 |
| 3 | 95.4 | 0.75 | 0.0 |
| 7 | 83.2 | 2.54 | 0.53 |
| 24 | 74.4 | 5.84 | 2.73 |

Control experiments using only AspFB24XylA or GsTpi for conversion of dihydroxyacetone into glyceraldehyde for the C-C coupling with fructose-6-phosphate aldolase or transaldolase B showed almost no accumulation (Table 25) compared to the other used hydroxypyruvate isomerases or 2-dehydrotetronate isomerase. The results are also shown in Fig. 1. All enzymes for isomerization of dihydroxyacetone were used at the same concentration of 1.2 mg/ml.

**Table 25: Comparison of different isomerases for conversion of dihydroxyacetone for production of D-fructose after 24**

| Enzyme | Fructose (mM) |
|---|---|
| *Asp*FB24XylA | **1.39** |
| *Gs*Tpi | **1.43** |
| *Ec*Hyi | **5.96** |
| *Cc*Hyi | **7.33** |
| *Pw*Hyi | **15.75** |
| *Hs*Hyi | **6.14** |
| *Pp*Hyi | **32.16** |
| *Vp*Hyi | **21.28** |
| *Vn*Hyi | **21.31** |
| *Cn*Hyi | **20.05** |
| *Sa*Hyi | **8.45** |
| *St*Hyi | **6.32** |
| *Ec*Otnl | **5.84** |

### Biotransformation of Dihydroxyacetone to Fructose/Glucose/Mannose

The cell-free bioproduction of fructose/glucose/mannose was performed in 1000 µl scale in Eppendorf tube. The solution contained the combination of enzymes as shown in Table. 10.0 mM MgCl₂ and 1.0 mM MnCl₂ and 1.0 mM TPP and 25-50 mM TRIS-HCl or HEPES pH 8.0. and 100 mM DHA at 25°C. The reaction was carried out in triplicates. The formation of fructose, glucose and mannose was followed over time by withdrawing 100 µl of aliquot at certain time intervals. The sample was diluted 25-fold using 5 mM H₂SO₄ prior to filtration through 10 KDa spin column (VWR. Germany). The filtrate was analyzed by HPLC using an ion-exclusion column (RezexROA-Organic Acid H+(8%. Phenomenex. Germany) run isocratically using 2.5 mM H₂SO₄ at 70 °C for 20 min.

**Table 26: Enzymes used for biotransformation of dihydroxyacetone to D-fructose/D-glucose/D-mannose**

| Enzyme | Protein concentration (mg/ml) |
|---|---|
| PpHyi | 1.2 |
| *Ec*TalB-F178YR181E | 2.5 |
| *Asp*FB24XylA | 0.5 |
| *Ec*YihS | 1.0 |

**Table 27: Conversion of dihydroxyacetone to D-fructose/D-glucose/D-mannose**

| Time (h) | Dihydroxyacetone (mM) | D-Fructose (mM) | D-Glucose (mM) | D-Mannose (mM) |
|---|---|---|---|---|
| 0 | 98.3 | 0.0 | 0.0 | 0.0 |
| 3 | 90.2 | 7.2 | 0.64 | 0.21 |
| 7 | 76.4 | 13.4 | 4.7 | 2.6 |
| 24 | 10.3 | 25.3 | 12.4 | 6.3 |

### Biotransformation of Dihydroxyacetone to Ethanol

The cell-free bioproduction of ethanol was performed in 1000 µl scale in Eppendorf tube. The solution contained the combination of enzymes as shown in Table 28. 1.0 mM NAD⁺ and 10.0 mM MgCl₂ and 0.1 mM MnCl₂ and 1.0 mM TPP and 25-50 mM TRIS-HCl or HEPES pH 8.0. and 100 mM DHAat25°C. The reaction was carried out in triplicates. The formation of ethanol was followed over time by withdrawing 100 µl of aliquot at certain time intervals.

The sample was diluted 25-fold using 5 mM H₂SO₄ prior to filtration through 10 KDa spin column (VWR. Germany). The filtrate was analyzed by HPLC using an ion-exclusion column (RezexROA-Organic Acid H+(8%. Phenomenex. Germany) run isocratically using 2.5 mM H₂SO₄ at 70 °C for 20 min.

**Table 28: Enzymes used for biotransformation of dihydroxyacetone to ethanol**

| Enzyme | Protein concentration (mg/ml) |
|---|---|
| PpHyi | 1.2 |
| *Vp*ALDH | 2.5 |
| *Pu*DHT | 0.5 |
| *Ap*PDC | 1.0 |
| *Bs*ADH | 0.5 |

**Table 29: Conversion of dihydroxyacetone to ethanol**

| Time (h) | Dihydroxyacetone (mM) | Ethanol (mM) |
|---|---|---|
| 0 | 99.6 | 0.0 |
| 3 | 96.8 | 2.4 |
| 7 | 91.3 | 6.9 |
| 24 | 83.2 | 15.7 |

### Biotransformation of Dihydroxyacetone to L-Lactate

The cell-free bioproduction of lactate was performed in 1000 µl scale in Eppendorf tube. The solution contained the combination of enzymes as shown in Table 30. 1.0 mM NAD⁺ and 10.0 mM MgCl₂ and 0.1 mM MnCl₂ and 1.0 mM TPP and 100 mM TRIS-HCl or HEPES pH 8.0. and 100 mM DHA at 25°C. The reaction was carried out in triplicates. The formation of lactate was followed over time by withdrawing 100 µl of aliquot at certain time intervals.

The sample was diluted 25-fold using 5 mM H₂SO₄ prior to filtration through 10 KDa spin column (VWR. Germany). The filtrate was analyzed by HPLC using an ion-exclusion column (RezexROA-Organic Acid H+(8%. Phenomenex. Germany) run isocratically using 2.5 mM H₂SO₄ at 70 °C for 20 min.

**Table 30: Enzymes used for biotransformation of dihydroxyacetone to L-lactate**

| Enzyme | Protein concentration (mg/ml) |
|---|---|
| PpHyi | 1.2 |
| *Vp*ALDH | 2.5 |
| *Pu*DHT | 0.5 |
| *GsLDH* | 0.25 |

**Table 31: Conversion of dihydroxyacetone to L-lactate**

| Time (h) | Dihydroxyacetone (mM) | L-Lactate (mM) |
|---|---|---|
| 0 | 99.1 | 0.0 |
| 3 | 94.9 | 3.2 |
| 7 | 89.9 | 7.9 |
| 24 | 79.8 | 18.7 |

### Biotransformation of Dihydroxyacetone to L-Alanine

The cell-free bioproduction of alanine was performed in 1000 µl scale in Eppendorf tube. The solution contained the combination of enzymes as shown in Table 32. 1.0 mM NAD⁺ and 10.0 mM MgCl₂ and 0.1 mM MnCl₂ and 1.0 mM TPP and 100 mM TRIS-HCl or HEPES pH 8.0, 100 mM DHA and 200 mM NH₄Cl at 25°C. The reaction was carried out in triplicates. The formation of alanine was followed over time by withdrawing 100 µl of aliquot at certain time intervals.

The samples were analyzed by HPLC (Ultimate 3000 HPLC-system) system. For the quantification, derivatisation with OPA/3-MPA reagent was used, as recommended by the manufacturers of Dr. Maisch GmbH (Ammerbuch-Entringen, Germany). As column, a GromSil OPA-3, 3 µM, 125 mm × 4.0 mm from Dr. Maisch GmbH (Ammerbuch-Entringen, Germany) was used. Samples were diluted 1:10 or 1:20 in water and filtered using centrifugal filters modified PES 10 kDa (VWR). After filtration, 8 µl of sample were combined with 40 µl of 1 M borate buffer pH 10.7, 8 µl of I-norvaline (1.02 mM) as internal standard and 184 µl of water. For derivatisation and monitoring 14 µl of this mixture were mixed with 6 µl of OPA-reagent 1:10 diluted in 1 M borate buffer pH 10.7.

**Table 32: Enzymes used for biotransformation of dihydroxyacetone to L-alanine**

| Enzyme | Protein concentration (mg/ml) |
|---|---|
| PpHyi | 1.2 |
| *Vp*ALDH | 2.5 |
| *Pu*DHT | 0.5 |
| *BsALADH* | 0.5 |

**Table 33: Conversion of dihydroxyacetone to L-alanine**

| Time (h) | Dihydroxyacetone (mM) | L-Alanine (mM) |
|---|---|---|
| 0 | 98.7 | 0.0 |
| 3 | 94.7 | 3.5 |
| 7 | 91.2 | 7.1 |
| 24 | 77.8 | 21.2 |

### Biotransformation of Dihydroxyacetone to Isobutanol

The cell-free bioproduction of isobutanol was performed in 1000 µl scale in Eppendorf tube. The solution contained the combination of enzymes as shown in Table 34. 1.0 mM NAD⁺ and 10.0 mM MgCl₂ and 0.1 mM MnCl₂ and 1.0 mM TPP and 25-50 mM TRIS-HCl or HEPES pH 8.0. and 100 mM DHA at 25°C. The reaction was carried out in triplicates. The formation of isobutanol was followed over time by withdrawing 100 µl of aliquot at certain time intervals.

The sample was diluted 25-fold using 5 mM H₂SO₄ prior to filtration through 10 KDa spin column (VWR. Germany). The filtrate was analyzed by HPLC using an ion-exclusion column (RezexROA-Organic Acid H+(8%. Phenomenex. Germany) run isocratically using 2.5 mM H₂SO₄ at 70 °C for 20 min.

**Table 34: Enzymes used for biotransformation of dihydroxyacetone to isobutanol**

| Enzyme | Protein concentration (mg/ml) |
|---|---|
| PpHyi | 1.2 |
| *Vp*ALDH | 2.5 |
| *Pu*DHT | 0.5 |
| *MrKari* | 1.0 |
| *BsALS* | 1.0 |
| *BsADH* | 0.5 |

**Table 35: Conversion of dihydroxyacetone to isobutanol**

| Time (h) | Dihydroxyacetone (mM) | Isobutanol (mM) |
|---|---|---|
| 0 | 99.6 | 0.0 |
| 3 | 93.1 | 2.9 |
| 7 | 87.9 | 5.9 |
| 24 | 72.3 | 13.4 |

### Biotransformation of Dihydroxyacetone to L-Valine

The cell-free bioproduction of valine was performed in 1000 µl scale in Eppendorf tube. The solution contained the combination of enzymes as shown in Table 36. 1.0 mM NAD⁺ and 10.0 mM MgCl₂ and 0.1 mM MnCl₂ and 1.0 mM TPP and 100 mM TRIS-HCl or HEPES pH 8.0, 100 mM DHA and 200 mM NH₄Cl at 25°C. The reaction was carried out in triplicates. The formation of valine was followed over time by withdrawing 100 µl of aliquot at certain time intervals.

The samples were analyzed by HPLC (Ultimate 3000 HPLC-system) system. For the quantification, derivatisation with OPA/3-MPA reagent was used, as recommended by the manufacturers of Dr. Maisch GmbH (Ammerbuch-Entringen, Germany). As column, a GromSil OPA-3, 3 µM, 125 mm × 4.0 mm from Dr. Maisch GmbH (Ammerbuch-Entringen, Germany) was used. Samples were diluted 1:10 or 1:20 in water and filtered using centrifugal filters modified PES 10 kDa (VWR). After filtration, 8 µl of sample were combined with 40 µl of 1 M borate buffer pH 10.7, 8 µl of I-norvaline (1.02 mM) as internal standard and 184 µl of water. For derivatisation and monitoring 14 µl of this mixture were mixed with 6 µl of OPA-reagent 1:10 diluted in 1 M borate buffer pH 10.7.

**Table 36: Enzymes used for biotransformation of dihydroxyacetone to L-valine**

| Enzyme | Protein concentration (mg/ml) |
|---|---|
| PpHyi | 1.2 |
| *Vp*ALDH | 2.5 |
| *Pu*DHT | 0.5 |
| *BsALS* | 1.0 |
| *MrKARI* | 1.0 |
| *GsLEUDH* | 0.5 |

**Table 37: Conversion of dihydroxyacetone to L-valine**

| Time (h) | Dihydroxyacetone (mM) | L-Valine (mM) |
|---|---|---|
| 0 | 99.3 | 0.0 |
| 3 | 92.8 | 3.1 |
| 7 | 85.6 | 6.8 |
| 24 | 68.2 | 15.7 |

### Biotransformation of Dihydroxyacetone to D-Glycerate

The cell-free bioproduction of glycerate was performed in 1000 µl scale in Eppendorf tube under aerobic conditions. The solution contained the combination of enzymes as shown in Table 38. 10.0 mM MgCl₂ and 0.1 mM MnCl₂, 100 mM TRIS-HCl or HEPES pH 8.0 and 100 mM DHA at 25°C. The reaction was carried out in triplicates. The formation of glycerate was followed over time by withdrawing 100 µl of aliquot at certain time intervals.

The sample was diluted 25-fold using 5 mM H₂SO₄ prior to filtration through 10 KDa spin column (VWR. Germany). The filtrate was analyzed by HPLC using an ion-exclusion column (RezexROA-Organic Acid H+(8%. Phenomenex. Germany) run isocratically using 2.5 mM H₂SO₄ at 70 °C for 20 min.

**Table 38: Enzymes used for biotransformation of dihydroxyacetone to D-glycerate**

| Enzyme | Protein concentration (mg/ml) |
|---|---|
| CcHyi | 1.2 |
| *AldO* | 2.5 |
| *Catalase* | 0.02 |

**Table 39: Conversion of dihydroxyacetone to glycerate**

| Time (h) | Dihydroxyacetone (mM) | D-Glycerate (mM) |
|---|---|---|
| 0 | 99.3 | 0.0 |
| 3 | 92.1 | 6.4 |
| 7 | 82.1 | 16.3 |
| 24 | 60.2 | 35.2 |

### Biotransformation of Dihydroxyacetone to Pyruvate

The cell-free bioproduction of pyruvate was performed in 1000 µl scale in Eppendorf tube under aerobic conditions. The solution contained the combination of enzymes as shown in Table 40. and 100 mM TRIS-HCl or HEPES pH 8.0 and 100 mM DHA at 25°C. The reaction was carried out in triplicates. The formation of pyruvate was followed over time by withdrawing 100 µl of aliquot at certain time intervals.

The sample was diluted 25-fold using 5 mM H₂SO₄ prior to filtration through 10 KDa spin column (VWR. Germany). The filtrate was analyzed by HPLC using an ion-exclusion column (RezexROA-Organic Acid H+(8%. Phenomenex. Germany) run isocratically using 2.5 mM H₂SO₄ at 70 °C for 20 min.

**Table 40: Enzymes used for biotransformation of dihydroxyacetone to pyruvate**

| Enzyme | Protein concentration (mg/ml) |
|---|---|
| EcOtnI | 1.2 |
| *AldO* | 2.5 |
| *Catalase* | 0.02 |
| *PuDHT* | 0.5 |

**Table 41: Conversion of dihydroxyacetone to pyruvate**

| Time (h) | Dihydroxyacetone (mM) | Pyruvate (mM) |
|---|---|---|
| 0 | 99.6 | 0.0 |
| 3 | 95.4 | 3.6 |
| 7 | 90.2 | 7.8 |
| 24 | 88.3 | 10.3 |

As the previous examples show, the use of an appropriate isomerase for the conversion of DHA into GA allows the production of several valuable chemicals. Only by the use of an isomerase that is capable of the conversion of DHA into GA the processes allow the efficient conversion of DHA. The versatility of DHA as a substrate for the production of various chemicals can be tapped by its ability to provide glyceraldehyde for the further conversion steps. Combining these isomerases with further enzymatic steps allows to push the system towards the product site.

As can be derived from above the inventive process for converting dihydroxyacetone to glyceraldehyde is suitable for the application in multi-enzymatic reaction cascades. Besides, the process can be applied in the preparation of larger products that is the production of carbohydrates as well as smaller molecules such as ethanol. Identification of isomerases able to convert DHA into GA without phosphorylation allows the cell-free biocatalytic use of DHA as a promising substrate basis and the generation of a huge variety of valuable chemicals as products.

The conversion from DHA to GA being possible without phosphorylation is desirable and possible with the inventive process with good yields and good activity of the isomerases.

## Claims

1. A process for converting dihydroxyacetone into glyceraldehyde comprising the step of enzymatically converting dihydroxyacetone into glyceraldehyde using an isomerase that a) is capable of catalyzing the conversion of dihydroxyacetone into glyceraldehyde.

2. Process according to claim 1,
wherein the isomerase
a) is capable of catalyzing the conversion of dihydroxyacetone into glyceraldehyde; and
b) comprises alpha keto-acid isomerase activity, preferably beta hydroxy-alpha keto-acid isomerase activity.

3. Process according to claim 1 or 2,
wherein the isomerase is an isomerase belonging to the EC5.3.1 class.

4. Process according to any of the preceding claims,
wherein the isomerase is an isomerase belonging to the EC5.3.1.22 class or EC5.3.1.35 class.

5. Process according to any of the preceding claims,
wherein the isomerase is a hydroxypyruvate isomerase or a 2-dehydrotetronate isomerase; or
wherein the isomerase is a hydroxypyruvate isomerase comprising an amino acid sequence that is at least 70 %, preferably at least 80 % and more preferably at least 90 % identical with the sequence according to SEQ ID NO: 35; or
wherein the isomerase is a hydroxypyruvate isomerase having an amino acid sequence according to SEQ ID NO: 5, SEQ ID NO: 27, SEQ ID NO: 29, SEQ ID NO: 31, SEQ ID NO: 33, SEQ ID NO: 35, SEQ ID NO: 39, SEQ ID NO: 37, SEQ ID NO: 45, or SEQ ID NO: 47.

6. Process according to claims 1 to 4,
wherein the isomerase is a 2-dehydrotetronate isomerase; or
wherein the isomerase is a 2-dehydrotetronate isomerase comprising an amino acid sequence that is at least 70 %, preferably at least 80 % and more preferably at least 90 % identical with the sequence according to SEQ ID NO: 43; or
wherein the isomerase is a 2-dehydrotetronate isomerase having an amino acid sequence according to SEQ ID NO: 43.

7. Process according to any of the preceding claims,
wherein the isomerase is derived from an organism being selected from the group consisting of *Escherichia coli, Cohaesibacter celericrescens, Polynucleobacter wuianus, Sulfobacillus acidophilus, Sulfobacillus thermosulfidooxidans, Pseudomonas putida, Cupriavidus necator, Vibrio natriegens, Variovorax paradoxus or Herbaspirillum seropedicae;* or
wherein the isomerase is a hydroxypyruvate isomerase being derived from *Pseudomonas putida.*

8. Process according to any of the preceding claims,
wherein said dihydroxyacetone is in a non-phosphorylated form; and/or
wherein said process is cell-free.

9. An isomerase being capable of catalyzing the conversion of dihydroxyacetone into glyceraldehyde, preferably being capable of catalyzing the conversion of dihydroxyacetone into glyceraldehyde and comprising alpha keto-acid isomerase activity, preferably beta hydroxy-alpha keto-acid isomerase activity.

10. Isomerase according to claim 9,
wherein the isomerase comprises an amino acid sequence that is at least 70 %, preferably at least 80 %, more preferably at least 90 % and even more preferably at least 95 % identical with the sequence according to SEQ ID NO: 35 or SEQ ID NO: 43; and/or
wherein the isomerase is an isomerase belonging to the EC5.3.1 class, preferably the isomerase is an isomerase belonging to the EC5.3.1.22 class or EC5.3.1.35 class; and/or wherein the isomerase is a hydroxypyruvate isomerase or a 2-dehydrotetronate isomerase.

11. Isomerase according to claims 9 or 10, having an amino acid sequence according to SEQ ID NO: 5, SEQ ID NO: 27, SEQ ID NO: 29, SEQ ID NO: 31, SEQ ID NO: 33, SEQ ID NO: 35, SEQ ID NO: 39, SEQ ID NO: 37, SEQ ID NO: 43, SEQ ID NO: 45, or SEQ ID NO: 47.

12. Use of an isomerase being capable of catalyzing the conversion of dihydroxyacetone into glyceraldehyde, preferably being capable of catalyzing the conversion of dihydroxyacetone into glyceraldehyde, and comprising alpha keto-acid isomerase activity, preferably beta hydroxy-alpha keto-acid isomerase activity, in the conversion of dihydroxyacetone into glyceraldehyde.

13. Use of an isomerase being capable of catalyzing the conversion of dihydroxyacetone into glyceraldehyde, preferably being capable of catalyzing the conversion of dihydroxyacetone into glyceraldehyde, and comprising alpha keto-acid isomerase activity, preferably beta hydroxy-alpha keto-acid isomerase activity, in the conversion process of dihydroxyacetone to a carbohydrate, preferably fructose, glucose, mannose or a mixture thereof.

14. Use of an isomerase being capable of catalyzing the conversion of dihydroxyacetone into glyceraldehyde, preferably being capable of catalyzing the conversion of dihydroxyacetone into glyceraldehyde, and comprising alpha keto-acid isomerase activity, preferably beta hydroxy-alpha keto-acid isomerase activity, in the conversion process of dihydroxyacetone to ethanol, alanine, pyruvate, glycerate, valine, isobutanol, and/or lactate.

15. Use of an isomerase according to claims 12 to 14,
wherein the isomerase is an isomerase belonging to the EC5.3.1 class, preferably the isomerase is an isomerase belonging to the EC5.3.1.22 class or EC5.3.1.35 class; and/or wherein the isomerase is a hydroxypyruvate isomerase or a 2-dehydrotetronate isomerase.
